# EUROPEAN PATENT SPECIFICATION

(11) **EP 3 242 864 B1**
(45) Date of publication and mention of the grant of the patent: **14.08.2019**
(21) Application number: 16700124.7
(22) Date of filing: 08.01.2016
(51) Int. Cl.: C07C 41/56, C07C 43/303

(54) **PROCESS OF PRODUCTION OF SPECIFIC KETALS**
VERFAHREN ZUR HERSTELLUNG BESTIMMTER KETALE
PROCÉDÉ DE PRODUCTION DE CÉTALS SPÉCIFIQUES

(30) Priority: 09.01.2015 EP 15150621
(43) Date of publication of application: 15.11.2017
(73) Proprietor: DSM IP Assets B.V., 6411 TE Heerlen (NL)
(72) Inventor: BONRATH, Werner, 4303 Kaiseraugst (CH); CLAVEY, Thomas, 4303 Kaiseraugst (CH); GUMMIN, Ingolf, 4303 Kaiseraugst (CH); MASCARELLO, Francesco, 4303 Kaiseraugst (CH); SCHULZ, Peter, 4303 Kaiseraugst (CH); THOMANN, Michael, 4303 Kaiseraugst (CH); WASSERSCHEID, Peter, 4303 Kaiseraugst (CH)
(74) Representative: Kurt, Manfred
(86) International application number: PCT/EP2016/050254
(87) International publication number: WO 2016/110560

(56) References cited:
- CN-A- 101 544 628
- CN-A- 102 584 513
- CN-A- 102 701 926
- FANG ET AL: "A green procedure for the protection of carbonyls catalyzed by novel task-specific room-temperature ionic liquid", CATALYSIS COMMUNICATIONS, ELSEVIER SCIENCE, AMSTERDAM, NL, vol. 8, no. 10, 14 September 2007 (2007-09-14), pages 1463-1466, XP022243698, ISSN: 1566-7367, DOI: 10.1016/J.CATCOM.2006.12.019 cited in the application
- DATABASE CA [Online] CHEMICAL ABSTRACTS SERVICE, COLUMBUS, OHIO, US; 2008, LONG, JIN-XING ET AL: "Synthesis of acetals or ketals via reaction of aldehydes or ketones with alcohols using ionic liquids as catalysts", XP002741007, retrieved from STN Database accession no. 2008:858659 & LONG, JIN-XING ET AL: "Synthesis of acetals or ketals via reaction of aldehydes or ketones with alcohols using ionic liquids as catalysts", FENZI CUIHUA , 22(3), 199-204 CODEN: FECUEN; ISSN: 1001-3555, 2008,

## Description

The present invention relates to a new method to produce specific acetals and ketals.

As it is known, acetals and ketals can be prepared by reacting an aldehyde or ketone with an alcohol in the presence of an acidic catalyst.

Therefore, the goal of the present invention was to find a process, which allows the production of specific acetals and ketals which is a simplified reaction process, and which does not have the disadvantages as listed above.

The acetals and ketals, which are produced by the process according to the present invention are the ones of formula (I) 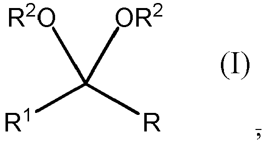 wherein
- R: is -CH₃, and
- R¹: is -CH₃ or -CH₂CH₃, and
- R²: is -CH₃,

These acetals and ketals, which are not cyclic and/or aromatic compounds, are useful compounds i.e. in the synthesis of other organic compound such as β-ionone, vitamin A or vitamin E.
Due to the importance of such acetals and ketals there is always a need for an improved process of production of such acetals and ketals.
The acetals and ketals (compounds of formula (I)) are usually produced according to the following reaction scheme:

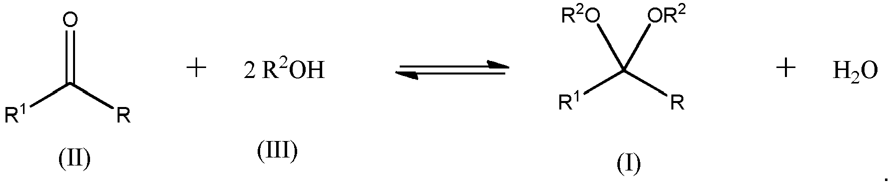

As stated above the reaction can easily be reversed, which needs to be avoided or at least minimized as good as possible. Otherwise the yield of the compound of formula (I) is very low and there is a need for extensive purification.

It is known from the prior art (i.e. US4136124) to produce 2,2-dimethoxypropane (R = R¹ = R² = CH₃ in formula (I)) by the reaction of acetone and methanol in the presence of HCl as a catalyst in a solvent (such decane). Especially the use of HCl is not desirable, because of the risk of corrosion.

In Green Chemistry 2006, 8, 1076 - 1079, Jiang et al. disclose and describe the synthesis of 2,2-dimethoxypropane in an ammonium ionic liquid. They disclose a process wherein acetone is reacted with methanol in the presence of anhydrous calcium sulfate. The anhydrous calcium sulfate is used in equimolar amount in regard to methanol. The anhydrous calcium sulfate serves to "bind" the water and shift the equilibrium to the product side. But a large amount of anhydrous calcium sulfate has to be used, which are then part of the reaction solution.

Fang et al (Catalysis Communications 8 (2007), p. 1463 - 1466) discloses that certain acetals and ketones can be produced by using a specific ionic liquid at temperatures between room temperature and 80°C.

CN 101 544 628 A discloses a method for preparing ketals by catalysis of an ionic liquid.

Now the goal of the present invention relates was to find an improved way of producing these acetals and ketals (compounds of formula (I)) without having the disadvantages of the prior art.

Surprisingly, it was found that the use of the specific ionic liquids (compounds of the formula (IV)) and the use of a low reaction temperature (below room temperature) allows to produce acetals and ketals (compounds of formula (I)) in excellent yield in the absence of HCI as well as in the absence of any anhydrous calcium sulfate (or compounds having the same purpose).

The used ionic liquid is the one of formula (IV) 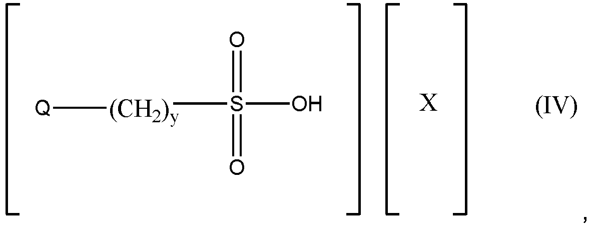 wherein
X is anion chosen from the group consisting of HSO₄⁻, H₂PO₄⁻, NO₃⁻, BF₄⁻, ClO₄⁻, CH₃SO₃⁻, CF₃SO₃⁻ and N(CmF₂ₘ₋₁)₂⁻, wherein m is 1, 2, 3, 4, 5, 6, 7, or 8;
y is 3 or 4, and
Q is either a moiety 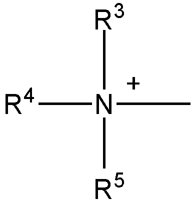 wherein
R³ is CH₃ or CH₂CH₃,
R⁴ is CH₃ or CH₂CH₃,
R⁵ is CH₃ or CH₂CH₃,
with the proviso that the total sum of the carbon atoms of the compound of formula (IV) is at least 6, preferably 6, 7, 8, 9, or 10;
or
Q is a moiety 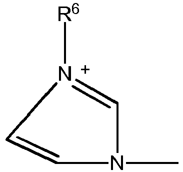 wherein
R⁶ is CH₃ or CH₂CH₃,
with the proviso that the total sum of the carbon atoms of the compound of formula (IV) is at least 6, preferably 6, 7, 8, 9, or 10.

Therefore the present invention relates to a process (P) for the production of compounds of formula (I) 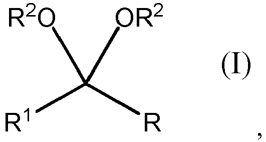 wherein
R is -CH₃, and
R¹ is -CH₃ or -CH₂CH₃, and
R² is -CH₃,
by reacting a compound of formula (II) 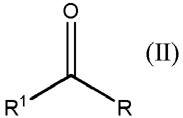 and a compound of formula (III)

R²OH (III),

wherein the R, R¹ and R² have the same meaning as in formula (I),
in at least one ionic liquid of formula (IV) 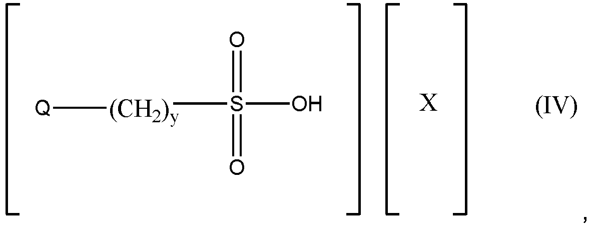 wherein
X is anion chosen from the group consisting of HSO₄⁻, H₂PO₄⁻, NO₃⁻, BF₄⁻, ClO₄⁻, CH₃SO₃⁻, CF₃SO₃⁻ and N(CmF₂ₘ₋₁)₂⁻, wherein m is 1, 2, 3, 4, 5, 6, 7, or 8;
y is 3 or 4, and
Q is either a moiety 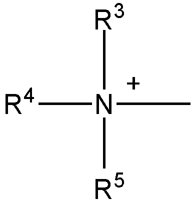 wherein
R³ is H, CH₃ or CH₂CH₃,
R⁴ is CH₃ or CH₂CH₃,
R⁵ is CH₃ or CH₂CH₃,
with the proviso that the total sum of the carbon atoms of the compound of formula (IV) is at least 6, preferably 6, 7, 8, 9, or 10;
or
Q is a moiety 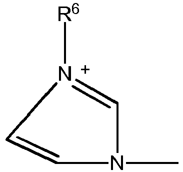 wherein
R⁶ is CH₃ or CH₂CH₃,
with the proviso that the total sum of the carbon atoms of the compound of formula (IV) is at least 6, preferably 6, 7, 8, 9, or 10, and
wherein the reaction temperature is between -60°C to 0°C.

It is surprising that the choice of a low reaction temperature has such an impact on the reaction. Comparisons examples have been carried out and can be found further below in the example part of the present patent application.

The molar ratio of the compound of formula (III) to the compound of formula (II) in the process according to the present invention is usually between 1:1 to 10:1. It is preferred to use an excess of the compound of formula (III).

Preferably the molar ratio of the compound of formula (III) to the compound of formula (II) in the process according to the present invention is between 1.5:1 to 8:1.

Therefore the present invention also relates to a process (P4), which is process (P), wherein the molar ratio of the compound of formula (III) to the compound of formula (II) is between 1:1 to 10:1, preferably between 1.5:1 to 8:1.

The amount of ionic liquid, which is used in the process according to the present invention can vary. It is possible to use one ionic liquid as well as a mixture of two or more ionic liquids. Preferably one ionic liquid is used.
It was found that surprisingly a relatively high amount (not catalytic amounts) of the ionic liquid leads to improved yields. Usually an ionic liquid is used in lower concentrations.

Usually at least 5 mol-%, based on the total mol of the compounds used in the process according to the present invention, are used. Usually not more than 90 mol-% are used.
Preferably the ionic liquid is used in an amount of 10 - 80 mol-%, based on the total mol of the compounds used in the process according to the present invention, preferably 10 - 80 mol-%, more preferably 10 - 70 mol-%.

Therefore the present invention also relates to a process (P5), which is process (P) or (P4), wherein at least 5 mol-%, based on the total mol of the compounds used in the process according to the present invention, of the at least one ionic liquid of formula (IV) is used, preferably 10-80 mol-%, more preferably 10 - 70 mol-%.

Preferred are processes wherein ionic liquids of formula (IV') are used 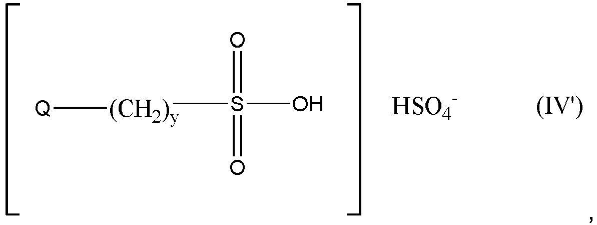 wherein
y is 3 or 4, and
Q is either a moiety 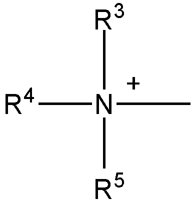 wherein
R³ is CH₃ or CH₂CH₃,
R⁴ is CH₃ or CH₂CH₃,
R⁵ is CH₃ or CH₂CH₃,
with the proviso that the total sum of the carbon atoms of the compound of formula (IV) is at least 6, preferably 6, 7, 8, 9, or 10;
or
Q is a moiety 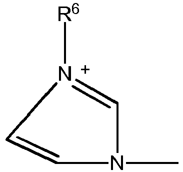 wherein
R⁶ is CH₃ or CH₂CH₃,
with the proviso that the total sum of the carbon atoms of the compound of formula (IV) is at least 6, preferably 6, 7, 8, 9, or 10.

Therefore the present invention also relates to a process (P6), which is process (P), (P4) or (P5), wherein ionic liquids of formula (IV') 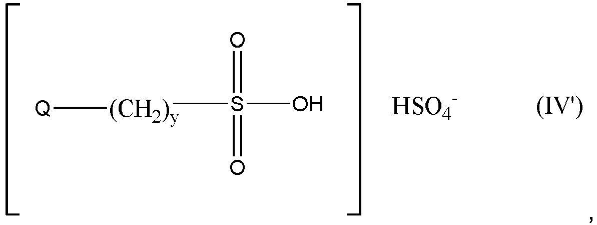 wherein
y is 3 or 4, and
Q is either a moiety 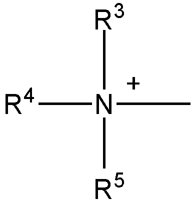 wherein
R³ is CH₃ or CH₂CH₃,
R⁴ is CH₃ or CH₂CH₃,
R⁵ is CH₃ or CH₂CH₃,
with the proviso that the total sum of the carbon atoms of the compound of formula (IV) is at least 6, preferably 6, 7, 8, 9, or 10;
or
Q is a moiety 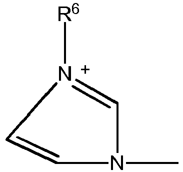 wherein
R⁶ is CH₃ or CH₂CH₃,
with the proviso that the total sum of the carbon atoms of the compound of formula (IV) is at least 6, preferably 6, 7, 8, 9, or 10,
are used.

More preferred ionic liquids used in the process according to the present invention are the ionic liquids of the following formulae (IVa) - (IVi) 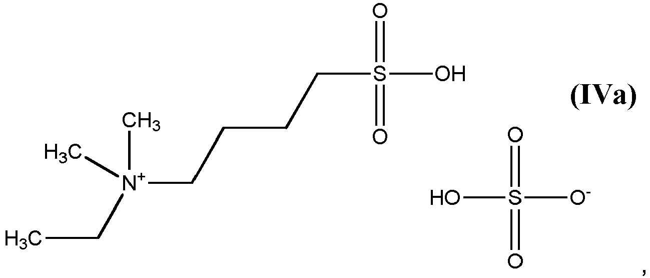 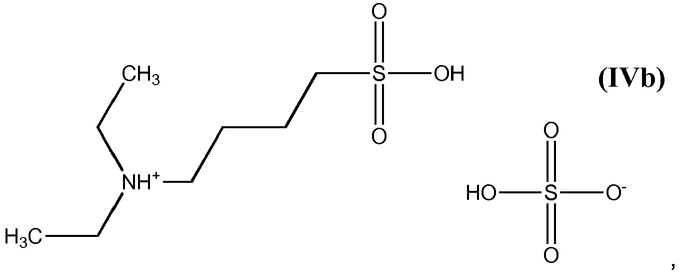 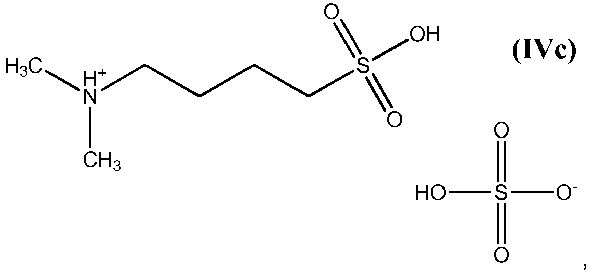 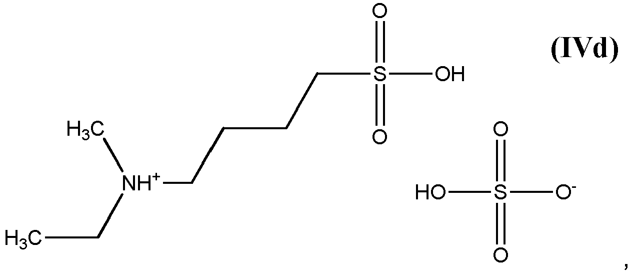 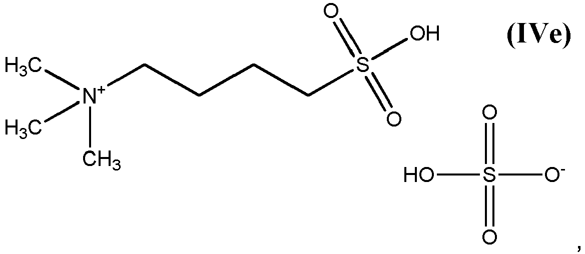 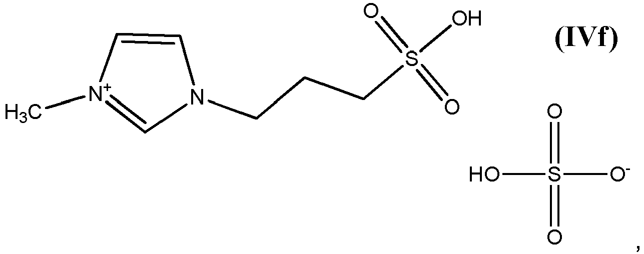 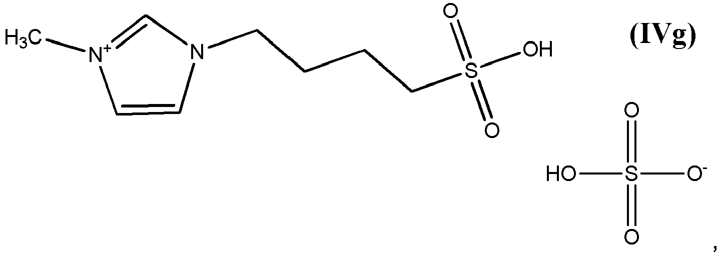 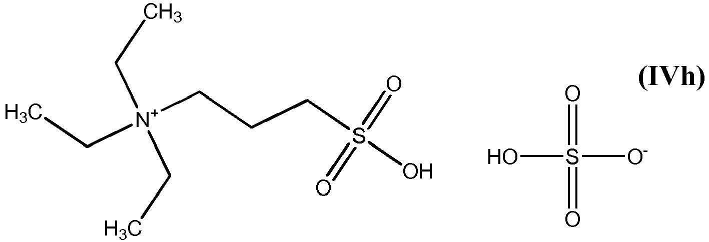 and

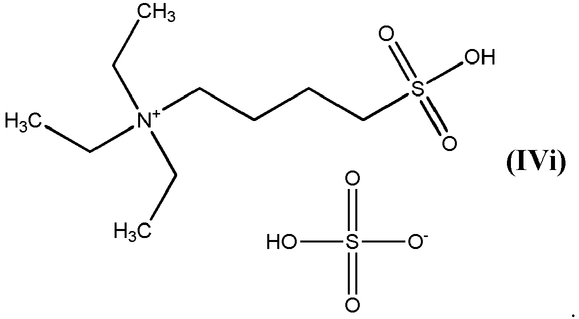

Therefore the present invention also relates to a process (P7), which is process (P), (P4), (P5) or (P6), wherein the ionic liquid is chosen from the group consisting of the ionic liquids of the following formulae (IVa) - (IVi) 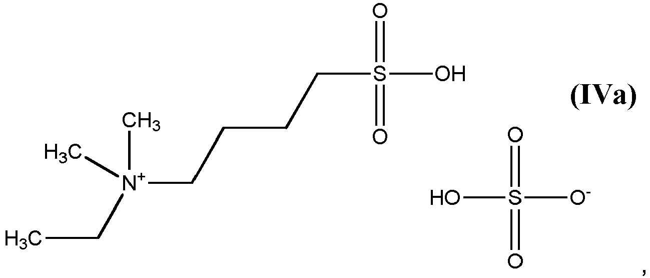 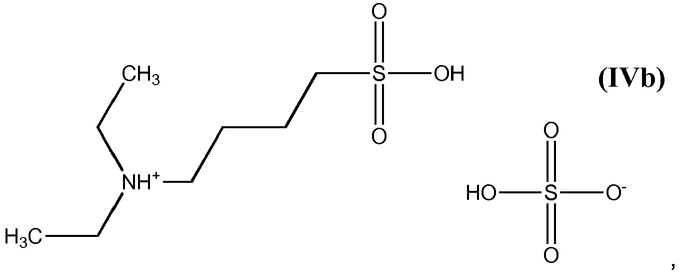 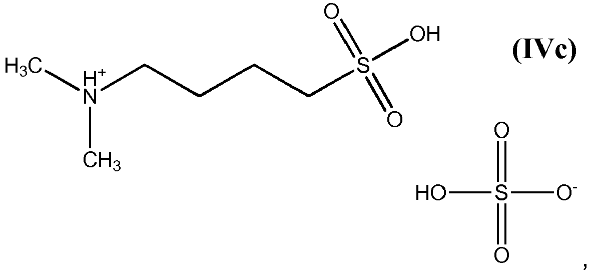 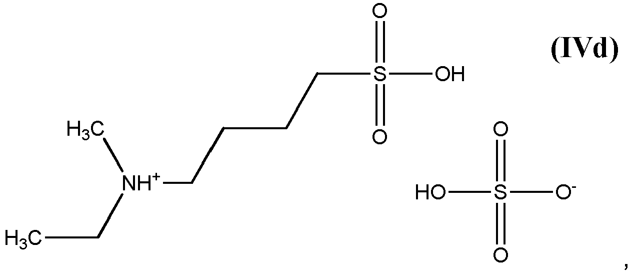 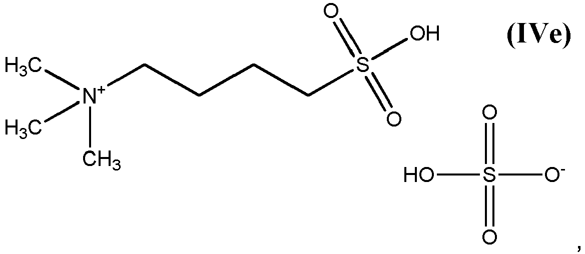 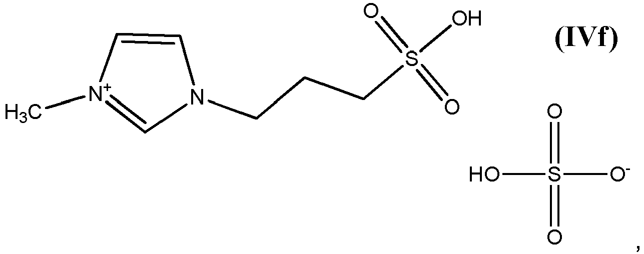 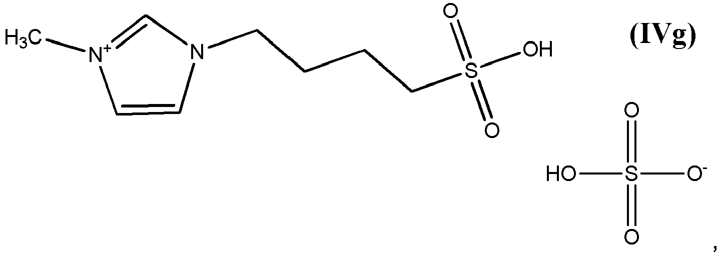 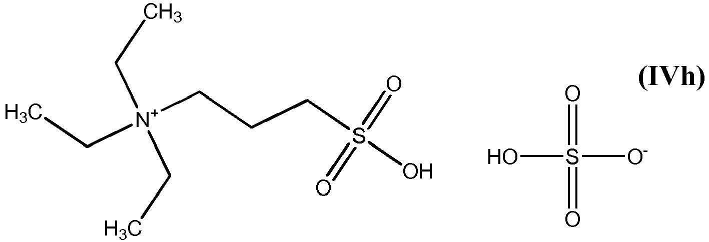 and

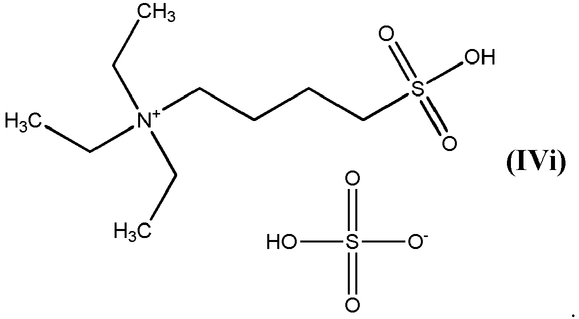

An essential feature of the present invention is the use of specific ionic liquid. Some of these ionic liquids are also novel.
Novel are the ionic liquids of formula (IV") 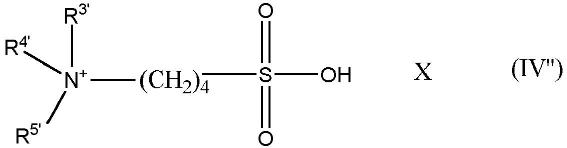 wherein
R^{3'} is H, -CH₃, and
R^{4'} is CH₃ or CH₂CH₃, and
R^{5'} is CH₃ or CH₂CH₃, and
X is anion chosen from the group consisting of HSO₄⁻, H₂PO₄⁻, NO₃⁻, BF₄⁻, ClO₄⁻, CH₃SO₃⁻, CF₃SO₃⁻ and N(CmF₂ₘ₋₁)₂⁻, wherein m is 1, 2, 3, 4, 5, 6, 7, or 8; preferably HSO₄⁻.

Preferred new ionic liquids are those of formula (IVa) - (IVd) 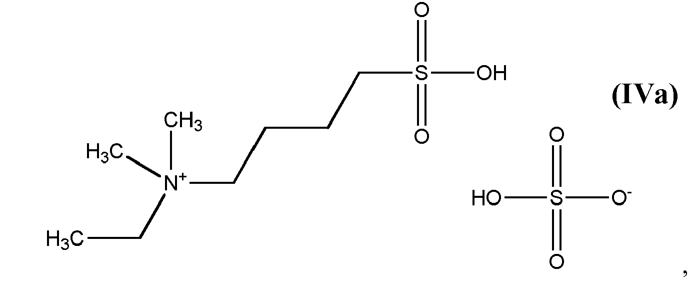 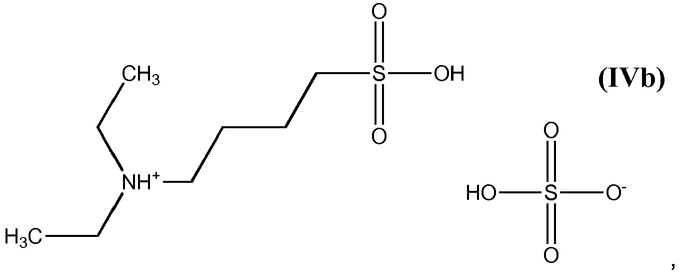 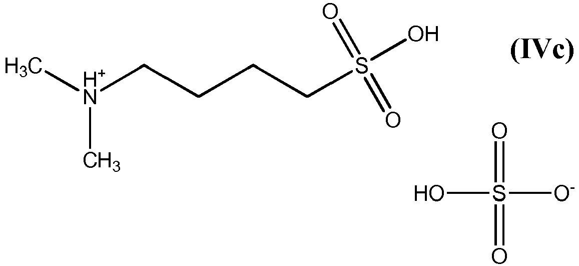 and

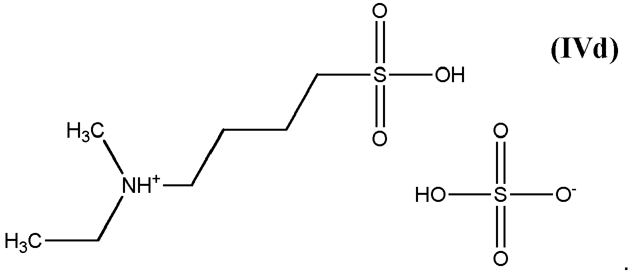

The (known) ionic liquids can be produced by commonly known processes or can be bought commercially.

The new ionic liquids are usually produced by the following process.
Firstly the zwitterion is produced by reacting a compound of formula (V) 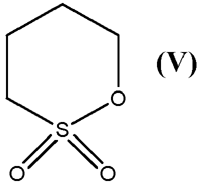 with a compound for formula (VI) 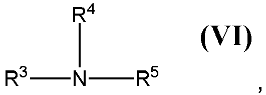 wherein
R^{3'} is H, -CH₃, and
R^{4'} is CH₃ or CH₂CH₃, and
R^{5'} is CH₃ or CH₂CH₃.
This reaction is usually carried out in a suitable solvent (such as acetonitrile) at elevated temperature (30°C - 100°C).
In a second step the obtained zwitterion is then mixed with sulfuric acid (at elevated temperature, usually 40°C - 100°C).
The ionic liquid is obtained in excellent yield.

The obtained product of formula (I) is removed from the reaction mixture by commonly known processes.

As stated above the compounds of formula (I) can be used as starting material for the production of other compounds (such as for example β-ionone, vitamin A or vitamin E).

The following examples serve to illustrate the invention. All percentages and parts (if not otherwise stated) are related to the weight and the temperature is given in °C.

### Examples

### Synthesis of the ionic liquids (synthesis examples 1 to 4 are reference examples)

### Example 1: Dimethyl-ethyl-(4-sulfobutyl)ammonium hydrogensulfate ([DMEABS][HSO₄]); compound formula (IVa)

At first, the zwitterion dimethyl-ethyl-(4-sulfonatobutyl)ammonium (DMEABS) was synthesized by adding 1,4-butane sultone (67.48 g, 496 mmol, 50.36 ml) to a solution of dimethylethylamine (32.55 g, 445 mmol, 48.23 ml) in acetonitrile (150 ml) at 80 °C. The reaction mixture was then stirred for 15 h at 80 °C. The formed precipitation was separated by filtration and subsequently washed with toluene and diethyl ether. The white solid was finally dried in vacuum to yield pure DMEABS as a white powder. The yield of DMEABS was 95.35 %.

The zwitterion DMEABS (40 g, 191 mmol) was mixed with sulfuric acid (18.74 g, 191 mmol). After stirring for 2 h at 80 °C, the formed [DMEABS][HSO₄] was dried in medium vacuum and obtained in 100 % yield as colorless liquid.

### Example 2: Diethyl-(4-sulfobutyl)ammonium hydrogensulfate ([DEABS][HSO₄]); compound of formula (IVb)

At first, the zwitterion diethyl-(4-sulfonatobutyl)ammonium (DEABS) was synthesized by adding 1,4-butane sultone (34.04 g, 250 mmol, 25.4 ml) to a solution of diethylamine (18.29 g, 250 mmol, 26.12 ml) in acetonitrile (600 ml) at 50 °C. The reaction mixture was then stirred for 15 h at 50 °C. The formed precipitation was separated by filtration and subsequently washed with toluene and diethyl ether. The white solid was finally dried in vacuum to yield pure DEABS as a white powder. The yield of DEABS was 23.41 %.

The zwitterion DEABS (16 g, 76 mmol) was mixed with sulfuric acid (7.5 g, 76 mmol). After stirring for 2 h at 80 °C, the formed [DEABS][HSO₄] was dried in medium vacuum and obtained in 100 % yield as colorless liquid.

### Example 3: Dimethyl-(4-sulfobutyl)ammonium hydrogensulfate ([DMABS][HSO₄]; compound of formula (IVc)

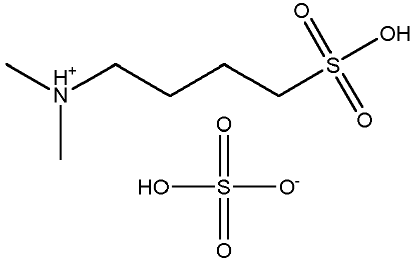

At first, the zwitterion dimethyl-(4-sulfonatobutyl)ammonium (DMABS) was synthesized by adding 1,4-butane sultone (51.06 g, 375 mmol, 38.1 ml) to a solution of dimethylamine (33 wt% in ethanol, 16.91 g, 375 mmol, 25.23 ml) in acetonitrile (900 ml) at 50 °C. The reaction mixture was then stirred for 15 h at 50 °C. The formed precipitation was separated by filtration and subsequently washed with toluene and diethyl ether. The white solid was finally dried in vacuum to yield pure DMABS as a white powder. The yield of DMABS was 76.12 %.

The zwitterion DMABS (23.07 g, 127 mmol) was mixed with sulfuric acid (12.48 g, 127 mmol). After stirring for 2 h at 80 °C, the formed [DMABS][HSO₄] was dried in medium vacuum and obtained in 100 % yield as colorless liquid.

### Example 4: Ethyl-methyl-(4-sulfobutyl)ammonium hydrogensulfate ([EMABS][HSO₄]); compound of formula (IVd)

At first, the zwitterion ethyl-methyl-(4-sulfonatobutyl)ammonium (EMABS) was synthesized by adding 1,4-butane sultone (51.06 g, 375 mmol, 38.1 ml) to a solution of ethylmethylamine (22.17 g, 375 mmol, 32.22 ml) in acetonitrile (900 ml) at 50 °C. The reaction mixture was then stirred for 15 h at 50 °C. The formed precipitation was separated by filtration and subsequently washed with toluene and diethyl ether. The white solid was finally dried in vacuum to yield pure EMABS as a white powder. The yield of EMABS was 17.62 %.

The zwitterion EMABS (11.8 g, 60 mmol) was mixed with sulfuric acid (5.93 g, 60 mmol). After stirring for 2 h at 80 °C, the formed [EMABS][HSO₄] was dried in medium vacuum and obtained in 100 % yield as colorless liquid.

### Synthesis of 2,2-dimethoxypropane

### Example 5: Synthesis of 2,2,-dimethoxypropane

8.71 g (31.18 mmol) of the ionic liquid of formula (IVe) 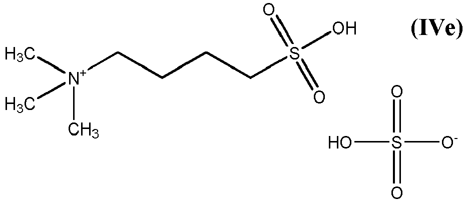 was dissolved in 14.39 g (449.04 mmol) methanol at room temperature. The obtained solution was cooled down to -50 °C.

Afterwards, 3.26 g (56.16 mmol) of acetone was added and the reaction mixture was stirred for 30 minutes at -50 °C. The stirrer was turned off. After 5 minutes the two-phase reaction system was analyzed by ¹H NMR spectroscopy. Using a syringe which contained 0.2 ml of pyridine, 0.2 ml of the upper product phase were sampled and dissolved in 0.2 ml of deuterated dimethyl sulfoxide. Using a syringe, 0.2 ml of the lower phase were sampled and dissolved in deuterated water. Both samples were analyzed by ¹H NMR spectroscopy. The content of 2,2-dimethoxypropane (DMP) in the upper phase was 70 wt% (3.5 g, 33.56 mmol). The yield of DMP was 59.8 %.

### Example 6: Synthesis of 2,2,-dimethoxypropane

11.38 g (37.63 mmol) of the ionic liquid of formula (IVf) 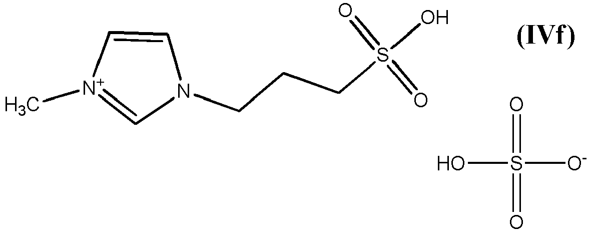 was dissolved in 4.58 g (142.9 mmol) methanol at room temperature. The obtained solution was cooled down to 0 °C.

Afterwards, 4.08 g (70.32 mmol) of acetone was added and the reaction mixture was stirred at 650 rpm for 30 minutes at 0 °C. The stirrer was turned off. After 5 minutes the two-phase reaction system was analyzed by ¹H NMR spectroscopy. Using a syringe which contained 0.2 ml of pyridine, 0.2 ml of the upper product phase were sampled and dissolved in 0.2 ml of deuterated dimethyl sulfoxide. Using a syringe, 0.2 ml of the lower phase were sampled and dissolved in deuterated water. Both samples were analyzed by ¹H NMR spectroscopy. The content of 2,2-dimethoxypropane (DMP) in the upper phase was 33 wt% (1.4 g, 13.42 mmol). The yield of DMP was 19.1 %.

### Example 7: Synthesis of 2,2,-dimethoxypropane

10 g (31.61 mmol) of the ionic liquid of formula (IVg) 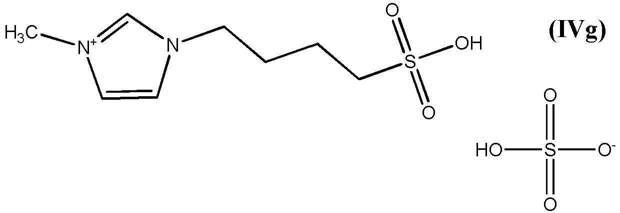 was dissolved in 2.46 g (76.65 mmol) methanol at room temperature. The obtained solution was cooled down to -18 °C.

Afterwards, 1.09 g (18.74 mmol) of acetone was added and the reaction mixture was stirred for 30 minutes at -18 °C. The stirrer was turned off. After 5 minutes the two-phase reaction system was analyzed by ¹H NMR spectroscopy. Using a syringe which contained 0.2 ml of pyridine, 0.2 ml of the upper product phase were sampled and dissolved in 0.2 ml of deuterated dimethyl sulfoxide. Using a syringe, 0.2 ml of the lower phase were sampled and dissolved in deuterated water. Both samples were analyzed by ¹H NMR spectroscopy. The content of 2,2-dimethoxypropane (DMP) in the upper phase was 56 wt% (0.57 g, 5.48 mmol). The yield of DMP was 29.3 %.

### Comparison tests

The following examples show the surprising and significant improvements of the process according to the present invention (especially when compared to prior art processes such as the one from Fang et al (Catalysis Communications 8 (2007), p. 1463 - 1466)).

### Comparison test series 1

Examples 1 - 4 in Table 1 have been carried out according to the following reaction process:
0.32 mmol of the ionic liquid of formula 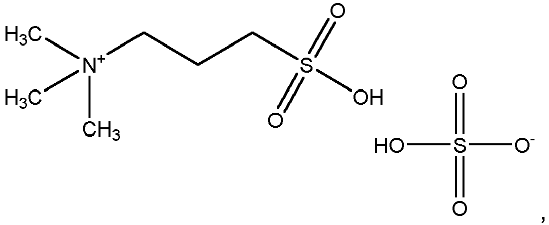 which is [TMPSA][HSO₄] as described and used in Fang et al (Catalysis Communications 8 (2007), p. 1463 - 1466),
was mixed with 30 mmol methanol at various temperatures (s. Table 1). Precipitation of the ionic liquid was observed for all examples.
Afterwards, 10 mmol of acetone was added and the reaction mixture was stirred for 15 minutes at various temperature (s. Table 1). The stirrer was turned off. The reaction system was analyzed by ¹H NMR spectroscopy. Using a syringe which contained 0.2 ml of pyridine, 0.2 ml of the reaction solution were sampled and dissolved in 0.2 ml of deuterated dimethyl sulfoxide. The sample was analyzed by ¹H NMR spectroscopy.

Examples 5 - 10 in Table 1 have been carried out according to the following reaction process:
0.2 mmol of the ionic liquid of formula 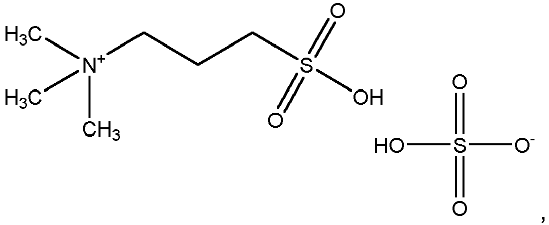 which is [TMPSA][HSO₄] as described and used in Fang et al (Catalysis Communications 8 (2007), p. 1463 - 1466),
was mixed with 15 mmol methanol at various temperatures (s. Table 1). Precipitation of the ionic liquid was observed.
Afterwards, 10 mmol of acetone was added and the reaction mixture was stirred for 15 minutes at various temperatures (s. Table 1). The stirrer was turned off. The reaction system was analyzed by ¹H NMR spectroscopy. Using a syringe which contained 0.2 ml of pyridine, 0.2 ml of the reaction solution were sampled and dissolved in 0.2 ml of deuterated dimethyl sulfoxide. The sample was analyzed by ¹H NMR spectroscopy.

The results are summarized in the following Table 1.

**Table 1**

| **Exp.** | **Ketone** | **Molar ratio** | **T (°C)** | **Yield of ketal (%)** | **Selectivity (%)** |
|---|---|---|---|---|---|
| 1 | Acetone | 1:3 | 25 | 12.7 | 100 |
| 2 | 2-Butanone | 1:3 | 25 | 7.3 | 100 |
| **3** | **Acetone** | **1:3** | **-18** | **21.1** | **100** |
| **4** | **2-Butanone** | **1:3** | **-18** | **13.5** | **100** |
| 5 | Acetone | 1:1.5 | 25 | 10.6 | 100 |
| 6 | 2-Butanone | 1:1.5 | 25 | 6.2 | 100 |
| **7** | **Acetone** | **1:1.5** | **-18** | **17.9** | **100** |
| **8** | **2-Butanone** | **1:1.5** | **-18** | **13.5** | **100** |
| **9** | **Acetone** | **1:1.5** | **-40** | **25.7** | **100** |
| **10** | **2-Butanone** | **1:1.5** | **-40** | **18.4** | **100** |

Examples 3, 4 and 7 - 10 are examples according to the present invention and show significantly improved yields, when compared with the prior art examples.

### Comparison test series 2

Examples 11 - 14 in Table 2 have been carried out according to the following reaction process:
0.32 mmol of the ionic liquid of formula 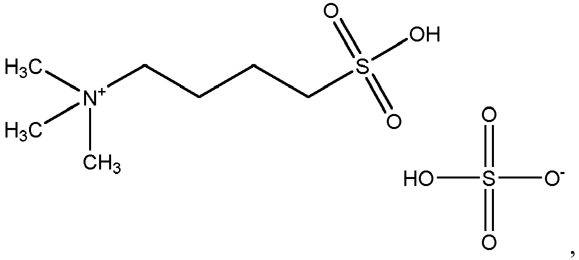 which is the ionic liquid [TMBSA][HSO₄] (ionic liquid of formula (IVe) of the present patent application),
was mixed with 30 mmol methanol at various temperatures (s. Table 2). Afterwards, 10 mmol of acetone was added and the reaction mixture was stirred for 15 minutes at various temperature (s. Table 2). The stirrer was turned off. The reaction system was analyzed by ¹H NMR spectroscopy. Using a syringe which contained 0.2 ml of pyridine, 0.2 ml of the reaction solution were sampled and dissolved in 0.2 ml of deuterated dimethyl sulfoxide. The sample was analyzed by ¹H NMR spectroscopy.

Examples 15 - 20 in Table 2 have been carried out according to the following reaction process:
0.2 mmol of the ionic liquid of formula 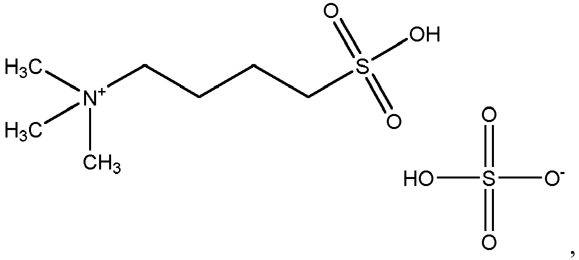 which is the ionic liquid [TMBSA][HSO₄] (ionic liquid of formula (IVe) of the present patent application),
was mixed with 15 mmol methanol at various temperatures (s. Table 2). Afterwards, 10 mmol of acetone was added and the reaction mixture was stirred for 15 minutes at various temperatures (s. Table 2). The stirrer was turned off. The reaction system was analyzed by ¹H NMR spectroscopy. Using a syringe which contained 0.2 ml of pyridine, 0.2 ml of the reaction solution were sampled and dissolved in 0.2 ml of deuterated dimethyl sulfoxide. The sample was analyzed by ¹H NMR spectroscopy. The results are summarized in the following Table 2.

**Table 2:**

| **Exp** | **Ketone** | **Molar ratio** | **T (°C)** | **Yield of ketal (%)** | **Selectivity (%)** |
|---|---|---|---|---|---|
| 11 | Acetone | 1:3 | 25. | 11.9 | 100 |
| 12 | 2-Butanone | 1:3 | 25 | 7.4 | 100 |
| **13** | **Acetone** | **1:3** | **-18** | **20.6** | **100** |
| **14** | **2-Butanone** | **1:3** | **-18** | **13.7** | **100** |
| 15 | Acetone | 1:1.5 | 25 | 10.4 | 100 |
| 16 | 2-Butanone | 1:1.5 | 25 | 6.3 | 100 |
| **17** | **Acetone** | **1:1.5** | **-18** | **17.7** | **100** |
| **18** | **2-Butanone** | **1:1.5** | **-18** | **13.7** | **100** |
| **19** | **Acetone** | **1:1.5** | **-40** | **25.3** | **100** |
| **20** | **2-Butanone** | **1:1.5** | **-40** | **18.7** | **100** |

Examples 13, 14 and 17 - 20 are examples according to the present invention and show significantly improved yields, when compared with the prior art examples.

### Comparison test series 3

Examples 21 - 24 in Table 3 have been carried out according to the following general reaction process:
4.46 mmol of the ionic liquid of formula 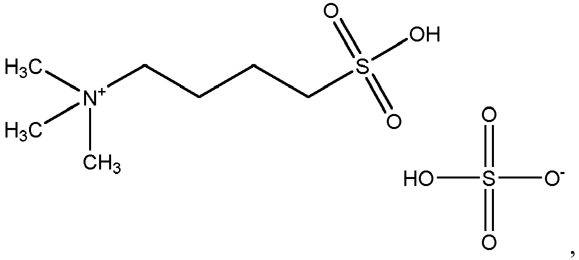 which is the ionic liquid [TMBSA][HSO₄] (ionic liquid of formula (IVe) of the present patent application),
was mixed with 30 mmol methanol at various temperatures (s. Table 3). Afterwards, 10 mmol of acetone was added and the reaction mixture was stirred for 15 minutes at various temperature (s. Table 3). The stirrer was turned off. The reaction system was analyzed by ¹H NMR spectroscopy. Using a syringe which contained 0.2 ml of pyridine, 0.2 ml of the reaction solution were sampled and dissolved in 0.2 ml of deuterated dimethyl sulfoxide. The sample was analyzed by ¹H NMR spectroscopy.

Examples 25 - 28 in Table 3 have been carried out according to the following general reaction process.
2.79 mmol of the ionic liquid of formula 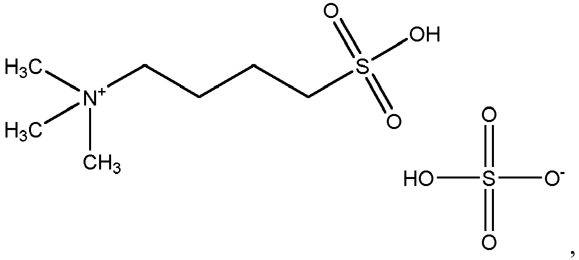 which is the ionic liquid [TMBSA][HSO₄] (ionic liquid of formula (IVe) of the present patent application),
was mixed with 15 mmol methanol at various temperatures (s. Table 3). Afterwards, 10 mmol of acetone was added and the reaction mixture was stirred for 15 minutes at various temperatures (s. Table 3). The stirrer was turned off. The reaction system was analyzed by ¹H NMR spectroscopy. Using a syringe which contained 0.2 ml of pyridine, 0.2 ml of the reaction solution were sampled and dissolved in 0.2 ml of deuterated dimethyl sulfoxide. The sample was analyzed by ¹H NMR spectroscopy. The results are summarized in the following Table 3.

**Table 3:**

| **Exp.** | **Ketone** | **Molar ratio** | **T (°C)** | **Yield of ketal (%)** | **Selectivity (%)** |
|---|---|---|---|---|---|
| 21 | Acetone | 1:3 | -18 | 36.7 | 100 |
| 22 | 2-Butanone | 1:3 | -18 | 25.7 | 100 |
| 23 | Acetone | 1:3 | -40 | 43.6 | 100 |
| 24 | 2-Butanone | 1:3 | -40 | 40.1 | 100 |
| 25 | Acetone | 1:1.5 | -18 | 29.9 | 100 |
| 26 | 2-Butanone | 1:1.5 | -18 | 24.2 | 100 |
| 27 | Acetone | 1:1.5 | -40 | 43.7 | 100 |
| 28 | 2-Butanone | 1:1.5 | -40 | 39.5 | 100 |

It can be seen from the Examples 21 - 28 (all part of the present invention) that a lower temperature as well as a higher molar ratio as well as a high amount of ionic liquid has a positive effect on the yield.

## Claims

1. A process for the production of compounds of formula (I) 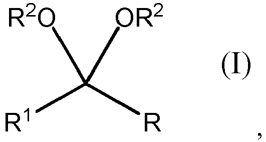 wherein
R is -CH₃, and
R¹ is -CH₃ or -CH₂CH₃, and
R² is -CH₃,
by reacting a compound of formula (II) 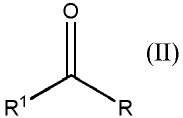 and a compound of formula (III)
R²OH (III),
wherein the R, R¹ and R² have the same meaning as in formula (I), in at least one ionic liquid of formula (IV) 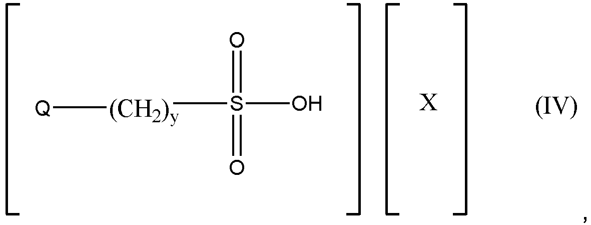 wherein
X is anion chosen from the group consisting of HSO₄⁻, H₂PO₄⁻, NO₃⁻, BF₄⁻, ClO₄⁻, CH₃SO₃⁻, CF₃SO₃⁻ and N(CmF₂ₘ₋₁)₂⁻, wherein m is 1, 2, 3, 4, 5, 6, 7, or 8;
y is 3 or 4, and
Q is either a moiety 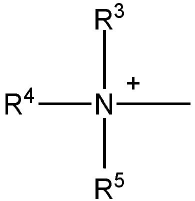 wherein
R³ is H, CH₃ or CH₂CH₃,
R⁴ is CH₃ or CH₂CH₃,
R⁵ is CH₃ or CH₂CH₃,
with the proviso that the total sum of the carbon atoms of the compound of formula (IV) is at least 6, preferably 6, 7, 8, 9, or 10;
or
Q is a moiety 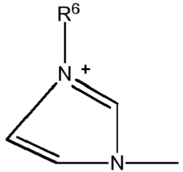 wherein
R⁶ is CH₃ or CH₂CH₃,
with the proviso that the total sum of the carbon atoms of the compound of formula (IV) is at least 6, preferably 6, 7, 8, 9, or 10 and
wherein the reaction temperature is between -60°C to 0°C.

2. Process according to claim 1, wherein the molar ratio of the compound of formula (III) to the compound of formula (II) is between 1:1 to 10:1, preferably between 1.5:1 to 8:1.

3. Process according to any of the preceding claims, wherein at least 5 mol-%, based on the total mol of the compounds used in the process according to the present invention, of the at least one ionic liquid of formula (IV) is used, preferably 10 - 80 mol-%.

4. Process according to any of the preceding claims, wherein ionic liquids of formula (IV') 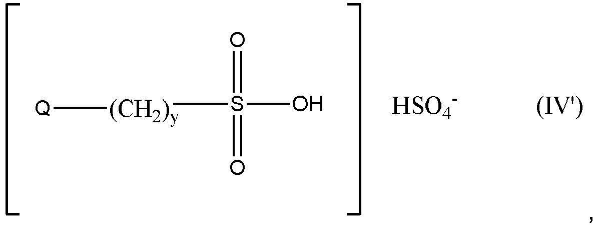 wherein
y is 3 or 4, and
Q is either a moiety 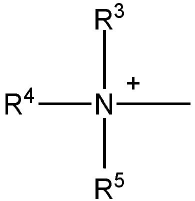 wherein
R³ is CH₃ or CH₂CH₃,
R⁴ is CH₃ or CH₂CH₃,
R⁵ is CH₃ or CH₂CH₃,
with the proviso that the total sum of the carbon atoms of the compound of formula (IV) is at least 6, preferably 6, 7, 8, 9, or 10;
or
Q is a moiety 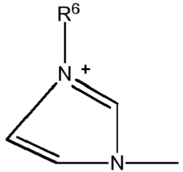 wherein
R⁶ is CH₃ or CH₂CH₃,
with the proviso that the total sum of the carbon atoms of the compound of formula (IV) is at least 6, preferably 6, 7, 8, 9, or 10,
are used.

5. Process according to any of the preceding claims, wherein the ionic liquid is chosen from the group consisting of the ionic liquids of the following formulae (IVa) - (IVi) 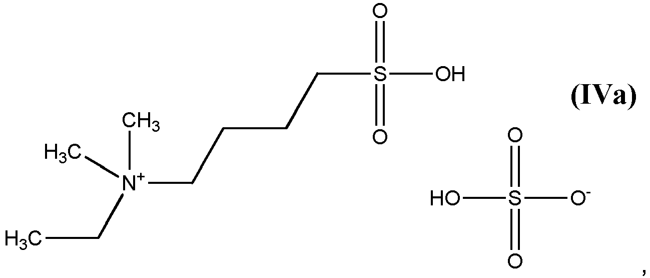 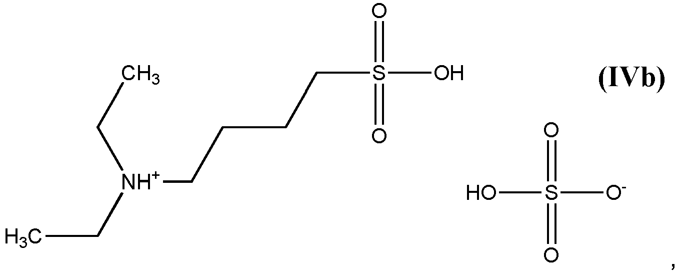 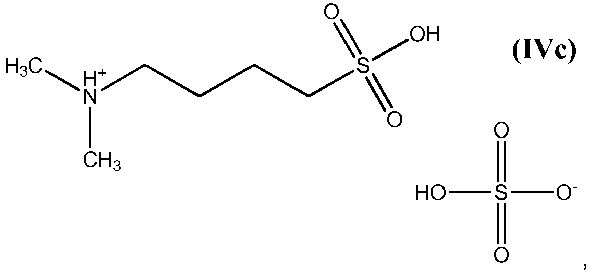 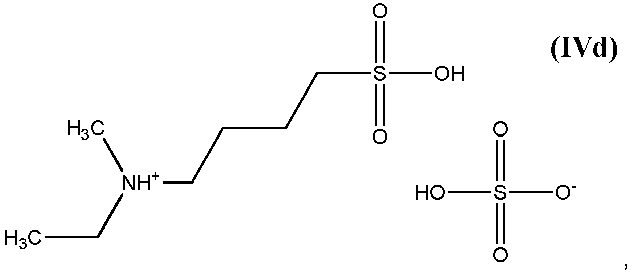 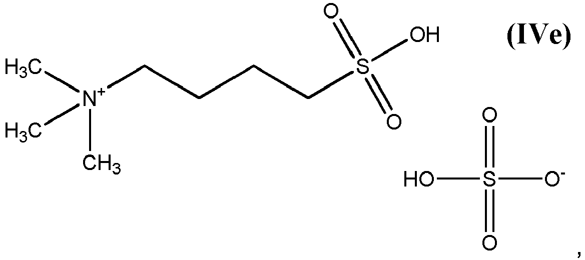 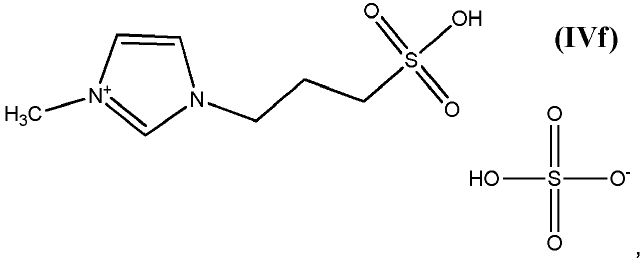 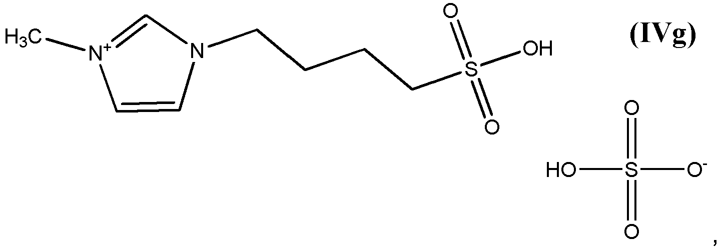 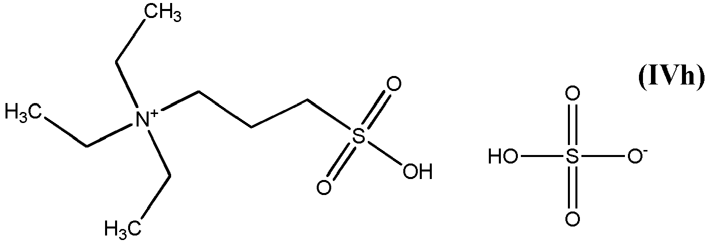 and

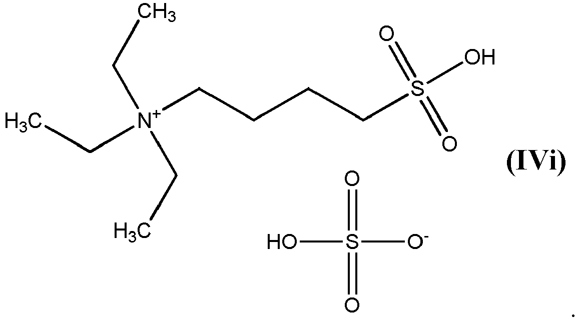

## Patentansprüche

1. Verfahren zur Herstellung von Verbindungen der Formel (I) 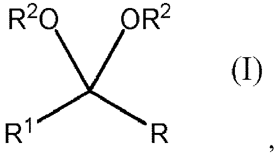 wobei
R für -CH₃ steht und
R¹ für -CH₃ oder -CH₂CH₃ steht und
R² für -CH₃ steht,
durch Umsetzung einer Verbindung der Formel (II) 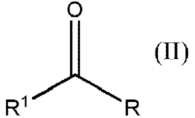 und einer Verbindung der Formel (III)
R²OH (III)
wobei R, R¹ und R² die gleiche Bedeutung wie in Formel (I) haben,
in mindestens einer ionischen Flüssigkeit der Formel (IV) 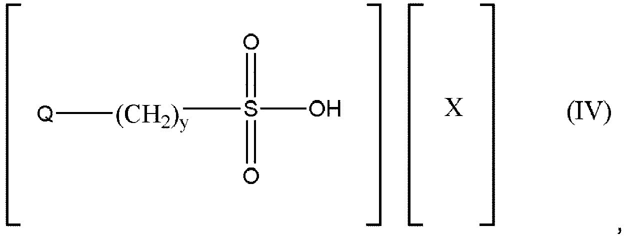 wobei
X für ein Anion aus der Gruppe bestehend aus HSO₄⁻, H₂PO₄⁻, NO₃⁻, BF₄⁻, ClO₄⁻, CH₃SO₃⁻, CF₃SO₃⁻ und N(CₘF₂ₘ₋₁)₂⁻ steht; wobei m für 1, 2, 3, 4, 5, 6, 7 oder 8 steht;
y für 3 oder 4 steht und
Q entweder für eine Gruppierung 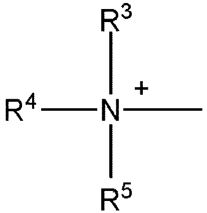 steht, wobei
R³ für H, CH₃ oder CH₂CH₃ steht,
R⁴ für CH₃ oder CH₂CH₃ steht,
R⁵ für CH₃ oder CH₂CH₃ steht,
mit der Maßgabe, dass die Gesamtsumme der Kohlenstoffatome der Verbindung der Formel (IV) mindestens 6, vorzugsweise 6, 7, 8, 9 oder 10 beträgt,
oder
Q für eine Gruppierung 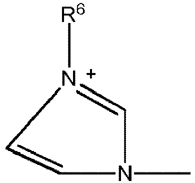 steht, wobei
R⁶ für CH₃ oder CH₂CH₃ steht,
mit der Maßgabe, dass die Gesamtsumme der Kohlenstoffatome der Verbindung der Formel (IV) mindestens 6, vorzugsweise 6, 7, 8, 9 oder 10 beträgt, und
wobei die Reaktionstemperatur zwischen -60 °C bis 0 °C liegt.

2. Verfahren nach Anspruch 1, bei dem das Molverhältnis von Verbindung der Formel (III) zu Verbindung der Formel (II) zwischen 1:1 bis 10:1, vorzugsweise zwischen 1,5:1 bis 8:1, liegt.

3. Verfahren nach einem der vorhergehenden Ansprüche, bei dem mindestens 5 Mol-%, bezogen auf die gesamten Mole der bei dem Verfahren gemäß der vorliegenden Erfindung verwendeten Verbindungen, der mindestens einen ionischen Flüssigkeit der Formel (IV) verwendet werden, vorzugsweise 10-80 Mol-%.

4. Verfahren nach einem der vorhergehenden Ansprüche, bei dem
ionische Flüssigkeiten der Formel (IV') 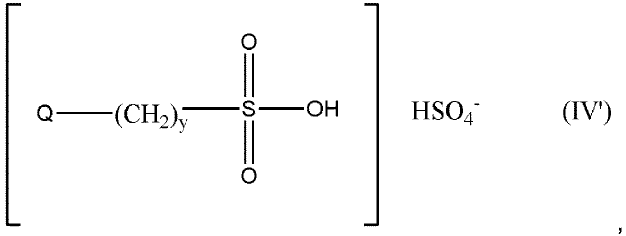 wobei
y für 3 oder 4 steht und
Q entweder für eine Gruppierung 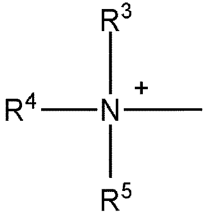 steht, wobei
R³ für H, CH₃ oder CH₂CH₃ steht,
R⁴ für CH₃ oder CH₂CH₃ steht,
R⁵ für CH₃ oder CH₂CH₃ steht,
mit der Maßgabe, dass die Gesamtsumme der Kohlenstoffatome der Verbindung der Formel (IV) mindestens 6, vorzugsweise 6, 7, 8, 9 oder 10 beträgt,
oder
Q für eine Gruppierung 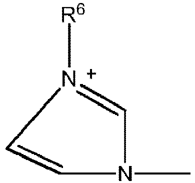 steht, wobei
R⁶ für CH₃ oder CH₂CH₃ steht,
mit der Maßgabe, dass die Gesamtsumme der Kohlenstoffatome der Verbindung der Formel (IV) mindestens 6, vorzugsweise 6, 7, 8, 9 oder 10 beträgt,
verwendet werden.

5. Verfahren nach einem der vorhergehenden Ansprüche, bei dem die ionische Flüssigkeit aus der Gruppe bestehend aus den ionischen Flüssigkeiten der folgenden Formeln (IVa)-(IVi) ausgewählt wird: 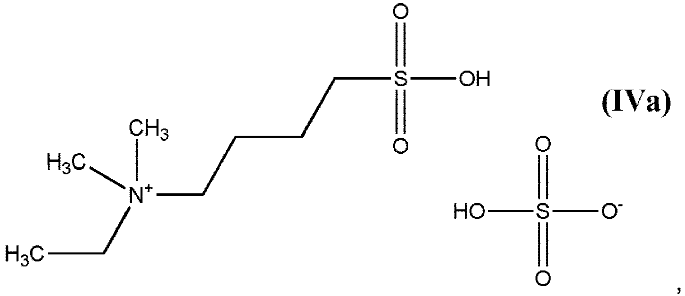 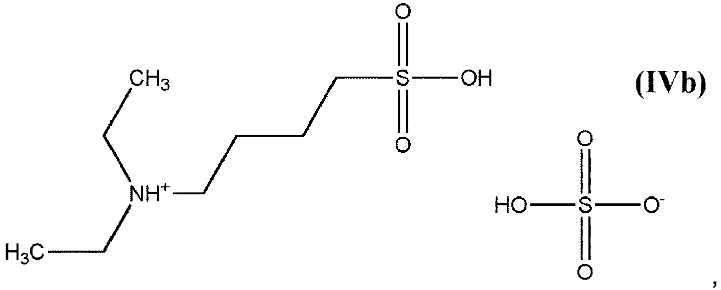 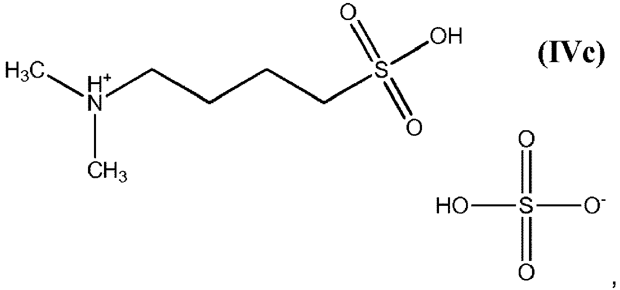 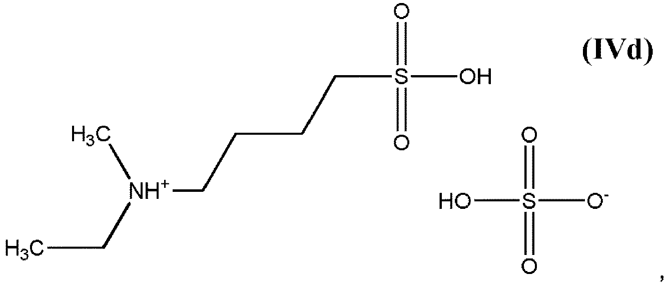 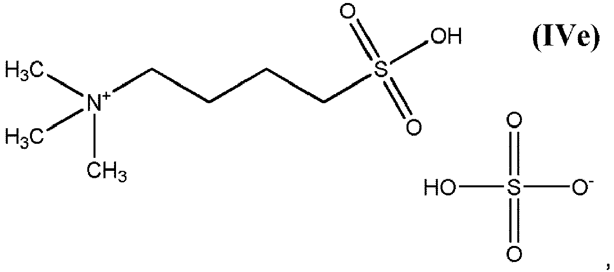 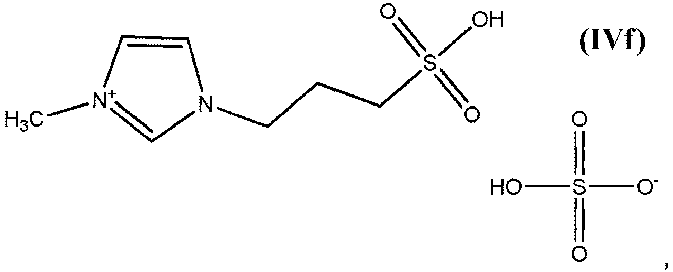 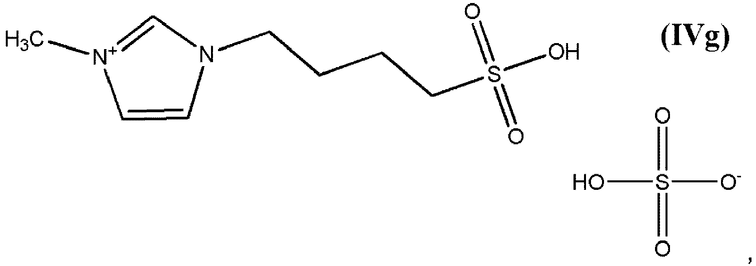 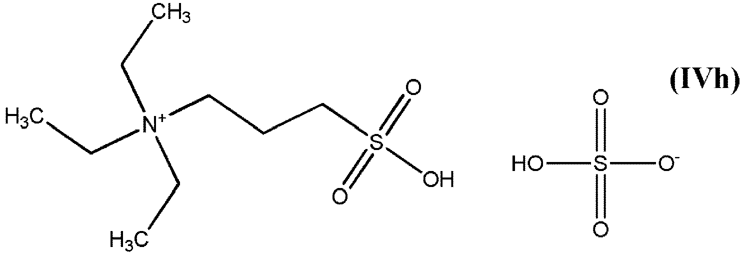 und

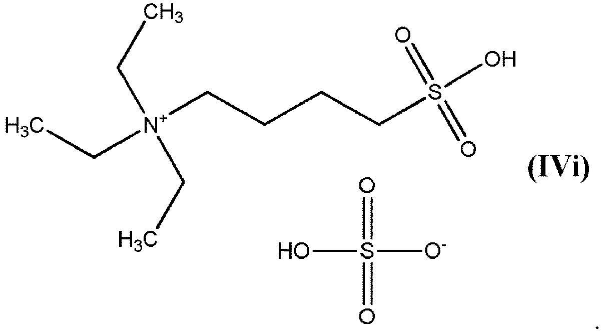

## Revendications

1. Procédé de production de composés de formule (I) 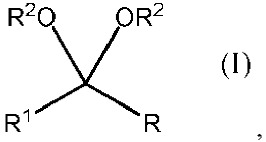 dans lequel
R est -CH₃, et
R¹ est -CH₃ ou -CH₂CH₃, et
R² est -CH₃,
par réaction d'un composé de formule (II) 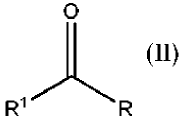 et d'un composé de formule (III)
R²OH (III),
dans lequel les R, R¹ et R² ont la même signification que dans la formule (I), dans au moins un liquide ionique de formule (IV) 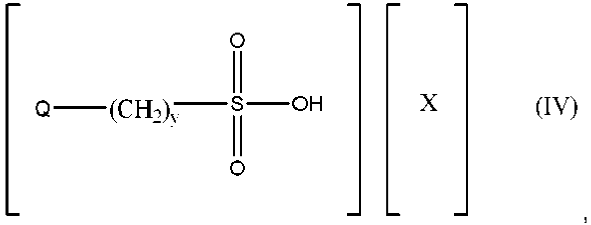 dans lequel
X est un anion choisi dans le groupe constitué de HSO₄⁻, H₂PO₄⁻, NO₃⁻, BF₄⁻, ClO₄⁻, CH₃SO₃⁻, CF₃SO₃⁻ et N(CₘF₂ₘ₋₁)₂⁻, dans lequel m est 1, 2, 3, 4, 5, 6, 7 ou 8 ;
y est 3 ou 4, et
Q est soit un fragment 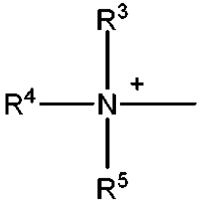 dans lequel
R³ est H, CH₃ ou CH₂CH₃,
R⁴ est CH₃ ou CH₂CH₃,
R⁵ est CH₃ ou CH₂CH₃,
à condition que la somme totale des atomes de carbone du composé de formule (IV) soit au moins 6, de préférence 6, 7, 8, 9 ou 10 ;
ou
Q est a fragment 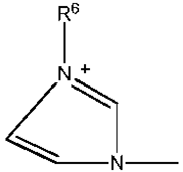 dans lequel
R⁶ est CH₃ ou CH₂CH₃,
à condition que la somme totale des atomes de carbone du composé de formule (IV) soit au moins 6, de préférence 6, 7, 8, 9 ou 10 et dans lequel la température de réaction est comprise entre -60 °C et 0 °C.

2. Procédé selon la revendication 1, dans lequel le rapport molaire du composé de formule (III) au composé de formule (II) est compris entre 1:1 et 10:1, de préférence entre 1,5:1 et 8:1.

3. Procédé selon l'une quelconque des revendications précédentes, dans lequel au moins 5 % en moles, sur la base des moles totales des composés utilisés dans le procédé selon la présente invention, de l'au moins un liquide ionique de formule (IV) est utilisé, de préférence 10 à 80 % en moles.

4. Procédé selon l'une quelconque des revendications précédentes, dans lequel
des liquides ioniques de formule (IV') 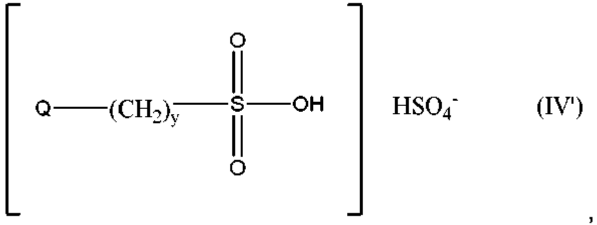 dans lequel
y est 3 ou 4, et
Q est un fragment 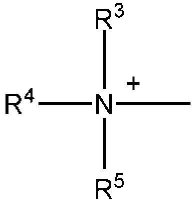 dans lequel
R³ est CH₃ ou CH₂CH₃,
R⁴ est CH₃ ou CH₂CH₃,
R⁵ est CH₃ ou CH₂CH₃,
à condition que la somme totale des atomes de carbone du composé de formule (IV) soit au moins 6, de préférence 6, 7, 8, 9 ou 10 ;
ou
Q est a fragment 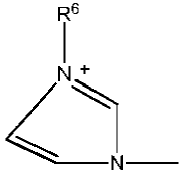 dans lequel
R⁶ est CH₃ ou CH₂CH₃, à condition que la somme totale des atomes de carbone du composé de formule (IV) soit au moins 6, de préférence 6, 7, 8, 9 ou 10,
sont utilisés.

5. Procédé selon l'une quelconque des revendications précédentes, dans lequel le liquide ionique est choisi dans le groupe constitué des liquides ioniques des formules (IVa) à (IVi) suivantes 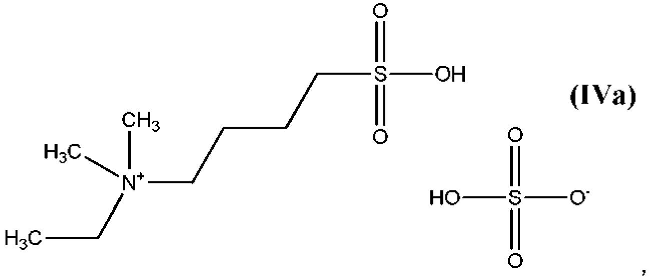 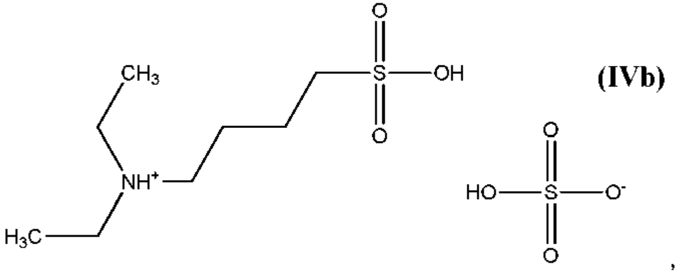 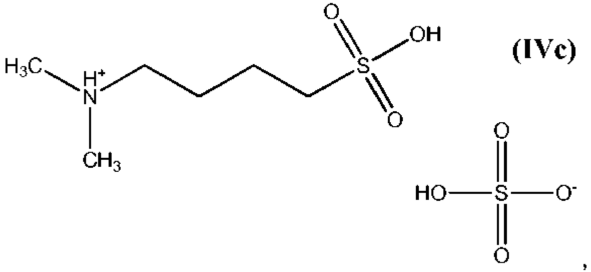 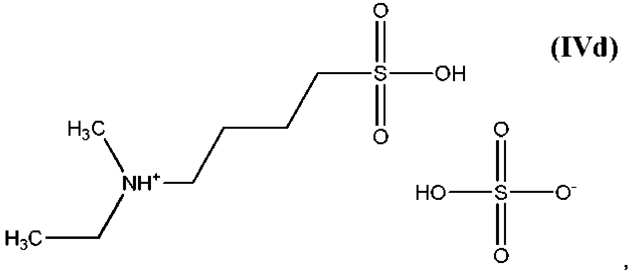 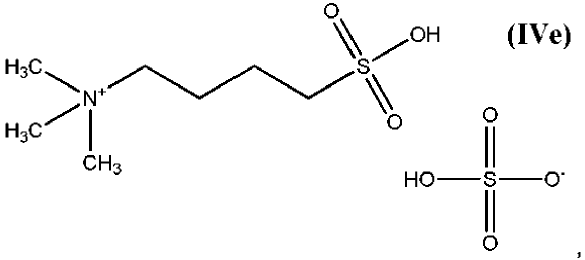 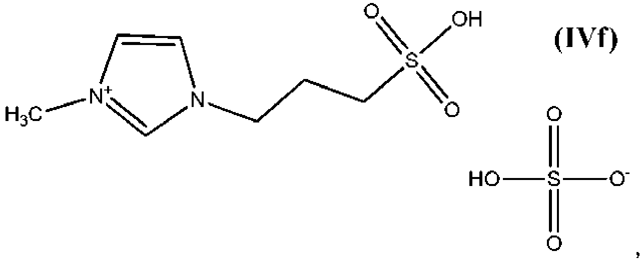 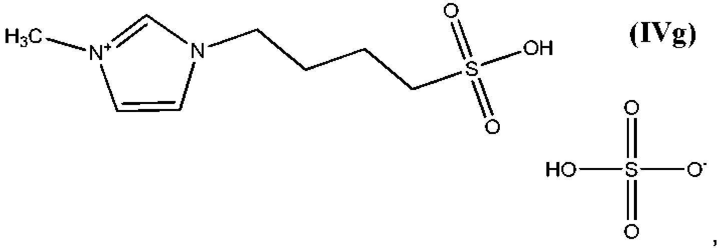 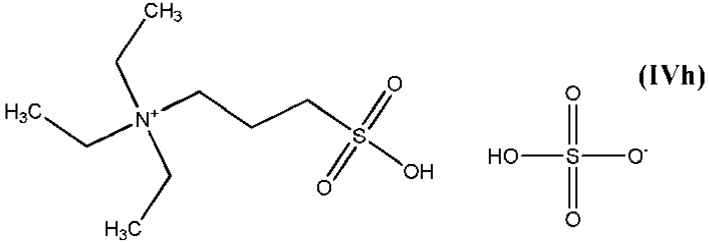 et
